# EUROPEAN PATENT SPECIFICATION

(11) **EP 1 749 031 B1**
(45) Date of publication and mention of the grant of the patent: **17.07.2013**
(21) Application number: 05748130.1
(22) Date of filing: 13.05.2005
(51) Int. Cl.: C07K 16/12, C07K 16/44, A61K 39/40

(54) **USE OF A scFv FOR DIMINISHING AN EXISTING BIOFILM**
VERWENDUNG VON EINEM scFv ZUR REDUZIERUNG VON EINEM BESTEHENDEN BIOFILM
UTILISATION D'UN scFv POUR REDUIRE UN BIOFILM EXISTANT

(30) Priority: 15.05.2004 GB 0410958
(43) Date of publication of application: 07.02.2007
(60) Divisional application: 10182405.0
(73) Proprietor: Haptogen Limited, Edinburgh Scotland EH2 3AH (GB)
(72) Inventor: CHARLTON, Keith, Foresterhill, Aberdeen AB25 2ZD (GB); PORTER, Andrew, Forrsterhill, Aberdeen AB25 2ZD (GB); THORNTHWAITE, Lorna, Foresterhill, Aberdeen AB25 2ZD (GB)
(74) Representative: Pfizer
(86) International application number: PCT/GB2005/001843
(87) International publication number: WO 2005/111080

(56) References cited:
- WO-A-2004/014423
- US-A1- 2003 095 985
- US-B1- 6 703 513
- HARTMAN G ET AL: "Quorum sensing: potential means of treating gram-negative infections?" LANCET THE, LANCET LIMITED. LONDON, GB, vol. 351, no. 9106, 21 March 1998 (1998-03-21), pages 848-849, XP004738315 ISSN: 0140-6736

## Description

### Fields of the invention

The present invention relates to methods for controlling and treating bacterial infections in patients. The invention provides for the application of therapies based upon, in the preferred embodiment, immunoglobulin or immunoglobulin-like receptor molecules that have affinity and specificity for acyl homoserine lactone signalling molecules involved in the processes of bacterial cell to cell communication. By binding to such molecules, the receptors can be used to modulate the extra-cellular concentrations of molecules involved in environment-sensing of bacteria, for example *Pseudomonas aeruginosa,* and/or other pathogenic bacteria, and in so doing can reduce or inhibit biofilm formation and virulence, and the associated resistance of biofilm bacteria to anti-bacterial agents.

### Background of the invention

One of the major causes of mortality and morbidity amongst patients undergoing treatment in hospitals today is due to hospital acquired infection. Susceptibility to such infection can be as a result of the primary illness for which the patient was admitted, of immuno-suppressive treatment regimes, or as a consequence of injury resulting in serious skin damage, such as bums. The bacterium to which the highest proportion of cases is attributed is *Pseudomonas aeruginosa.* It is the epitome of an opportunistic pathogen of humans. The bacterium almost never infects uncompromised tissues, yet there is hardly any tissue that it cannot infect, if the tissue defences are compromised in some manner. Although accounting for a relatively small number of species, it poses a serious threat to human health and is used hereafter as a representative example of an infectious bacterium, and does not in any way limit the scope or extent of the present invention.

*Ps. aeruginosa* is an opportunistic pathogen that causes urinary tract infections, respiratory system infections, dermatitis, soft tissue infections, bacteraemia and a variety of systemic infections, particularly in victims of severe bums, and in cancer and AIDS patients who are immuno-suppressed. Respiratory infections caused by *Ps. aeruginosa* occur almost exclusively in individuals with a compromised lower respiratory tract or a compromised systemic defence mechanism. Primary pneumonia occurs in patients with chronic lung disease and congestive heart failure. Bacteraemic pneumonia commonly occurs in neutropenic cancer patients undergoing chemotherapy. Lower respiratory tract colonisation of cystic fibrosis patients by mucoid strains of *Ps. aeruginosa* is common and difficult, if not impossible, to treat. It causes bacteraemia primarily in immuno-compromised patients. Predisposing conditions include haematologic malignancies, immuno-deficiency relating to AIDS, neutropenia, diabetes mellitus, and severe bums. Most *Pseudomonas* bacteraemia is acquired in hospitals and nursing homes where it accounts for about 25 percent of all hospital acquired gram-negative bacteraemias.

The bacterium is notorious for its natural resistant to many antibiotics due to the permeability barrier afforded by its outer membrane LPS and is, therefore, a particularly dangerous and dreaded pathogen. Also, its tendency to colonise surfaces in a biofilm form makes the cells impervious to therapeutic concentrations of antibiotics (Shih and Huang, 2002) and to host phagocytic cells (Wozniak et al., 2003). Biofilm formation is thought to play a key role in protecting bacteria from host defences. Studies have revealed that *Ps. aeruginosa* isolated from wounds are able to produce an exopolysaccharide capsule within a few hours of infection, a property that is likely to contribute significantly to successful colonisation (Harrisson-Baestra et. al., 2003). Since its natural habitat is the soil, living in association with the bacilli, actinomycetes and moulds, it has developed resistance to a variety of their naturally occurring antibiotics. Moreover, *Pseudomonas* spp. maintain antibiotic resistance plasmids, both Resistance factors (R-factors) and Resistance Transfer Factors (RTFs), and are able to transfer these genes by means of the bacterial processes of transduction and conjugation. Only a few antibiotics are effective against *Pseudomonas,* including fluoroquinolone, gentamicin and imipenem, and even these antibiotics are not effective against all strains. Combinations of gentamicin and carbenicillin are reportedly effective in patients with acute *Ps. aeruginosa* infections. The futility of treating *Pseudomonas* infections with antibiotics is most dramatically illustrated in cystic fibrosis patients, virtually all of whom eventually become infected with a strain that is so resistant it cannot be treated. Because of antibiotic resistance, susceptibility testing of clinical isolates is mandatory.

*Ps. aeruginosa* can usually be isolated from soil and water, as well as the surfaces of plants and animals. It is found throughout the world, wherever these habitats occur, so it is quite a "cosmopolitan" bacterium. It is sometimes present as part of the normal flora of humans, although the prevalence of colonisation of healthy individuals outside the hospital is relatively low (estimates range from 0 to 24 percent depending on the anatomical locale). In hospitals it is known to colonise food, sinks, taps, mops, respiratory equipment and surgical instruments. Although colonisation usually precedes infections by *Ps. aeruginosa,* the exact source and mode of transmission of the pathogen are often unclear because of its ubiquitous presence in the environment. Amongst intensive care patients in whom infection is suspected on clinical grounds, as many as 50% have no identifiable source for infection. Currently 1,400 deaths worldwide are caused each day by *Ps. aeruginosa* in intensive care units (ICU's), making it the No. 1 killer.

*Ps. aeruginosa* is primarily a nosocomial pathogen. According to the CDC, the overall incidence of *Ps. aeruginosa* infections in US hospitals averages about 0.4 percent (4 per 1000 discharges), and the bacterium is the fourth most commonly isolated nosocomial pathogen accounting for 10.1% of all hospital-acquired infections. Globally it is responsible for 16% of nosocomial pneumonia cases, 12% of acquired urinary tract infections, 8% of surgical wound infections and 10% of bloodstream infections. Immuno-compromised patients such as neutropenic cancer and bone marrow transplant patients are susceptible to opportunistic *Ps. aeruginosa* infection, leading to 30% of reported deaths. It is also responsible for 38% of ventilator-associated pneumonias and 50% of deaths amongst AIDS patients. In bums cases *Ps. aeruginosa* infections have declined in recent years due to improved treatment and dietary changes. Mortality rates however remain high, accounting for 60% all deaths due to secondary infection of bums patients.

One reason for the versatility of *Ps. aeruginosa* is that it produces a diverse battery of virulence determinants including elastase, LasA protease, alkaline protease, rhamnolipids, type IV pilus-mediated twitching motility, pyoverdin (Williams et al., 1996, Stintzi et al., 1998, Glessner et al., 1999), pyocyanin (Brint & Ohman, 1995, Reimmann et al., 1997) and the cytotoxic lectins PA-I and PA-II (Winzer et al., 2000). It is now known that many of these virulence determinants are regulated at the genetic level in a cell density-dependent manner through quorum sensing. Ps. *aeruginosa* possesses two well characterised quorum sensing systems, namely the *las* and *rhl (υsm)* systems which comprise of the LuxRI homologues LasRI (Gambello & Iglewski, 1991) and RHIRI (VsmRI) (Latifi et al., 1995) respectively. LasI directs the synthesis of 3-oxo-C12-HSL (Passador et al., 1993, Pearson et al., 1994) whereas RhII directs the synthesis of C4-HSL (Winson et al., 1995). The *las* and the *rhl* systems are thought to exist in a hierarchy where the *las* system exerts transcriptional control over RhIR (Williams et al., 1996, Pesci et al., 1997). The transcriptional activator LasR functions in conjunction with 3-oxo-C12-HSL to regulate the expression of the genes encoding for the virulence determinants elastase, LasA protease, alkaline protease and exotoxin A (Gambello & Iglewski, 1991, Toder et al., 1991, Gambello et al., 1993, Pearson et al., 1994) as well as *lasI.* Elastase is able to cleave collagen, IgG and IgA antibodies, complement, and facilitates bacterial adhesion onto lung mucosa. In combination with alkaline protease it also causes inactivation of gamma Interferon (INF) and Tumour Necrosis Factor (TNF). LasI directs the synthesis of 3-oxo-C12-HSL which together with LasR, binds to the *lasI* promoter and creates a positive feedback system. The RhIR transcriptional activator, along with its cognate AHL (C4-HSL), regulates the expression of *rhlAB* (rhamnolipid), *lasB, aprA,* RpoS, cyanide, pyocyanin and the lectins PA-I and PA-II (Ochsner et al., 1994, Brint & Ohman, 1995, Latifi et al., 1995, Pearson et al., 1995, Winson et al., 1995, Latifi et al., 1996, Winzer et al., 2000). These exist in a hierarchical manner where by the LasR/3-oxo-C12-HSL regulates *rhlR* (Latifi et al., 1996, Pesci et al., 1997) and consequently both systems are required for the regulation of all the above virulence determinants.

One of the most serious clinical conditions induced by *Ps. aeruginosa* is the destructive chronic lung infection of cystic fibrosis (CF) sufferers. Almost all patients' lungs are infected by the age of three years (Bums *et al.,* 2001). The immune systems of CF patients are unable to clear the bacteria, resulting in the onset of chronic disease with the associated extensive tissue damage and airway blockage from which the majority of patients eventually succumb. The establishment and persistence of *Ps. aeruginosa* lung infection has long been associated with the development of a biofilm phenotype, in addition to induction of other quorum-sensing regulated virulence factors (Singh *et al.,* 2000). Quorum sensing signals are readily detected in CF lung of infected mice (*Wu et al.,* 2000). Amongst other effects, the production of the well characterised AHL signalling molecules by *Ps. aeruginosa* in the lung can directly affect host immune responses by modulating the isotype ratio of the antibody response and cytokine levels (*Wu et al.,* 2004). Moreover, the growth of *Ps. aeruginosa* in biofilms results in very high cell densities of the order of 1 x 10¹⁰ cells/ml, the increased physical proximity of cells providing the perfect environment for enhanced cell-to-cell communication via quorum sensing and associated production of virulence factors.

A number of different approaches are being actively pursued to develop therapeutics for the treatment or prevention of *Ps. aeruginosa* infection. Some are intended to be broad ranging while others are directed at specific types of *Pseudomonas* infection. Those that follow traditional routes include the development of vaccines such as that described in US patent 6,309,651, and a new antibiotic drug (SLIT) that is hoped will be effective against gram-negative bacteria in general but is designed primarily to act against *Ps. aeruginosa* and is administered by aerosol inhalation. A further observation under investigation is that the antibiotic erythromycin administered at sub-optimal growth inhibitory concentrations simultaneously suppresses the production of *Ps. aeruginosa* haemagglutinins, haemolysin, proteases and acyl-homoserine lactones, and may be applicable for the treatment of persistent *Ps. aeruginosa* infection. Cream formulations containing amphipathic peptides are also being examined as a possible means of preventing infection of bums or other serious skin wounds. US patent 6,309,651 also teaches that antibodies against the PcrV virulence protein of *Ps. aeruginosa* may afford protection against infection.

There is also some interest in the modulation of homoserine lactone levels as a means of controlling pathogenicity. Certain algae have been demonstrated to produce competitive inhibitors of acyl-homoserine lactones such as furanones (Manefield, 1999), as have some terrestrial plants. These compounds displace the AHL signal molecule from its receptor protein and can act as agonist or antagonist in AHL bioassays (Tepletski et al., 2000). Other methods employed to reduce AHL concentration include the development of auto-inducer inactivation enzymes (AiiA's) that catalyse the degradation of AHLs and the sequestering of AHL by antibodies (WO 2004/014423). There has also been an increase in research into AHL mimics that compete with natural AHLs for receptor binding on the bacteria, but that do not trigger quorum-sensing regulated responses such as biofilm formation and virulence (Suga and Smith, 2003).

There are a number of potential problems and limitations associated with the therapies currently under development. It is as yet unproven as to whether vaccines will be efficacious treatments. *Ps. aeruginosa* produces an extensive mucoid capsule during biofilm growth that effectively protects against opsonisation by host antibodies, as revealed by patients with persistent infections having high serum titres of anti-*Pseudomonas* antibodies. This also provides the bacteria with significant protection against antibiotics and other anti-microbial chemicals. It has been demonstrated that clinical isolates of *Ps. aeruginosa* are resistant to elevated concentrations of antibiotics when growing as a biofilm, and that quorum-sensing defective mutants produce either less well developed or negligible polysaccharide and are killed by much lower antibiotic concentrations (Shih and Huang, 2002). The use of auto-inducer mimics are limited by the concentrations of most that are required to effectively compete against AHLs for the receptor binding site, and the possibility of side effects. It is well known that AHLs released by *Pseudomonas* and other bacteria have a number of direct effects on human physiology. These include inhibition of histamine release as described in WO 01/26650. WO 01/74801 describes that AHLs are also able to inhibit lymphocyte proliferation and down-regulate the secretion of TNF-α by monocytes and macrophages, so acting as a general immuno-suppressant. There is a danger therefore that therapies involving the use of competitive AHL mimics may result in down-regulation of the patient's immune system. This would be generally undesirable, and particularly so in immuno-compromised patients. The use of antibiotics can, at best, be viewed as a short-term strategy in view of the remarkable ability of this bacterium (and others) to develop resistance to specific antibiotics, and because of the general resilience to anti-microbial chemicals afforded by biofilm phenotype.

That the pathogenesis of *Ps. aeruginosa* is clearly multifactoral is underlined by the large number of virulence factors and the broad spectrum of diseases associated with this bacterium. Many of the extra-cellular virulence factors required for tissue invasion and dissemination, and the formation of biofilms, are controlled by cell-to-cell signalling systems involving homoserine lactone-based signal molecules and specific transcriptional activator proteins. These regulatory systems allow *Ps. aeruginosa* to adapt to a virulent form in a co-ordinated cell density dependent manner, and to overcome host defence mechanisms.

There is a need to develop effective means of modulating the concentrations of HSLs and other bacterial cell signalling molecules involved in pathogenicity by methods that do not have adverse side effects, and are unlikely to be evaded by pathogenic bacteria in the foreseeable future. A composition or compound capable of preventing biofilm formation in bacteria, particularly of *Pseudomonas aeruginosa,* that did not attack the bacterial cell directly and so is unlikely to lead to resistant strains would be of considerable benefit to the treatment of disease states such as CF and the prevention of wound infection. In particular, this would increase the effectiveness of many existing anti-microbial treatments and could make many of those that are no longer considered viable to be effective once again. The present invention provides for such compositions.

US patent application published as US 2003/0095985 describes immunogenic conjugates of gram-negative bacterial autoinducer molecules and antibodies raised against these molecules.

Also, US patent no. 6,703,513 describes production and use of derivatised homoserine lactones and antibodies to those molecules.

### Summary of the invention

The present invention provides for methods for reducing numbers of the pathogenic bacteria by regulating the extra-cellular concentrations of bacterial cell signalling molecules. By removal (binding or degradation) of lactone-derived cell signal molecules, the establishment of biofilms and biofilm-like growth could be inhibited, thereby increasing the susceptibility of pathogens to anti-microbial medicaments and to host defence mechanisms. Whereas other bactericidal treatments act directly on the cell to cause death, the present invention targets extra-cellular signalling molecules in order to reduce biofilm formation. As such it is much less likely that strains resistant to the therapy will emerge.

According to a first aspect of the invention, there is provided an antibody to the free form of a homoserine lactone signal molecule secreted by bacteria for use in diminishing an established biofilm produced by a population of bacteria, wherein the antibody is the single chain antibody (scAb) G3B12 deposited as NCIMB - 41168.

The lactone signal molecule may be a homoserine molecule or a peptide thiolactone molecule. The homoserine lactone molecule may have a general formula selected from the group consisting of: 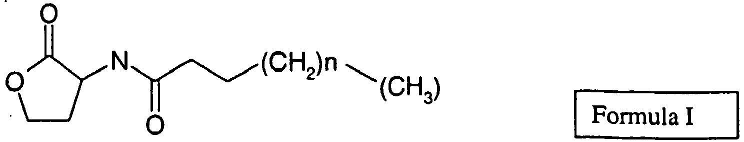 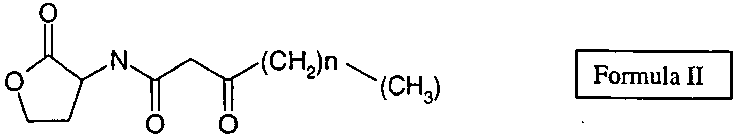 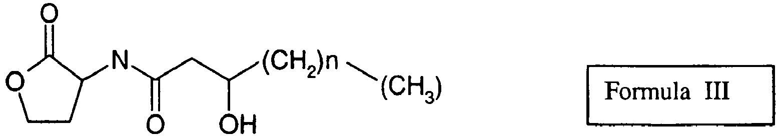 where n = 0 to 12..

The homoserine lactone molecule of general formula I may be N-butanoyl-L-homoserine lactone (BHL) where n = 0, N-dodecanoyl-L-homoserine lactone (dDHL) where n = 8 or n-tetradecanoyl-L-homoserine lactone (tDHL) where n = 10.

The homoserine lactone molecule of general formula II may be N-(-3-oxohexanoyl)-L-homoserine lactone (OHHL) where n = 2 or N-(-3-oxododecanoyl)-L-homoserine lactone (OdDHL) where n = 8.

The homoserine lactone molecule of general formula III may be N-(-3-hydroxybutanoyl)-L-homoserine lactone (HBHL) where n = 0.

The peptide thiolactone may have a general formula (IV) as follows: 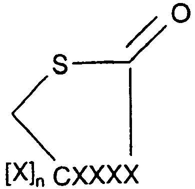 where X is any amino acid and n = 1 to 10.

Suitably, the peptide thiolactone molecule may be : 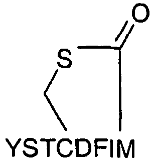 or 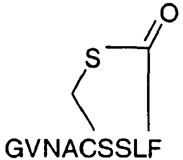 or 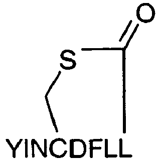 or

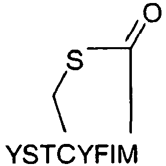

The lactone-derived signal molecule may also be a furanosyl borate diester. The furanosyl borate diester may be Auto Inducer-2 (AI-2),

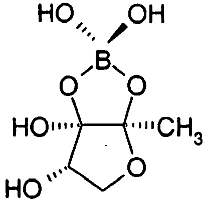

The lactone-derived signal molecule may also be Pro-AI-2 or a C₁-C₁₀ saturated or unsaturated carboxylic acid derivative thereof

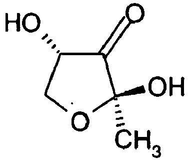

A growing number of bacterial species are being found to communicate between cells using a variety of small signal molecules. Gram-negative bacteria predominantly use N-acyl homoserine lactones. The latter are a group of compounds that share a common homoserine lactone ring structure and vary in the length and structure of a side chain. There are three classes within the group, the acyl-homoserine lactones, the 3-oxo-homoserine lactones and the 3-hydroxy-homoserine lactones. A single species can produce and respond to members of more than one class. *Pseudomonas aeruginosa* uses several, and particularly N-butyryl-homoserine lactone (BHL), 3-oxo-dodecanoyl-homoserine lactone (OdDHL) and to N-hexanoyl-homoserine lactone (HHL).

The cells use the molecules as a means of determining the local cell density, such that in conditions of low cell density the concentration of signal molecule is correspondingly low. In high cell densities the local signal molecule concentration is high. When this concentration reaches a threshold level it induces the transcription of genes involved in virulence and the onset of a disease state in the host. Many pathogenic bacteria including *Ps. aeruginosa* are able to grow as a biofilm. In such conditions, the cells are encased in an exopolysaccharide matrix. This both provides a physical barrier against anti-microbial agents and resistance to phagocytosis, but results in a high local cell density which leads to quorum-sensing induced switch of opportunistic bacteria to a pathogenic phenotype.

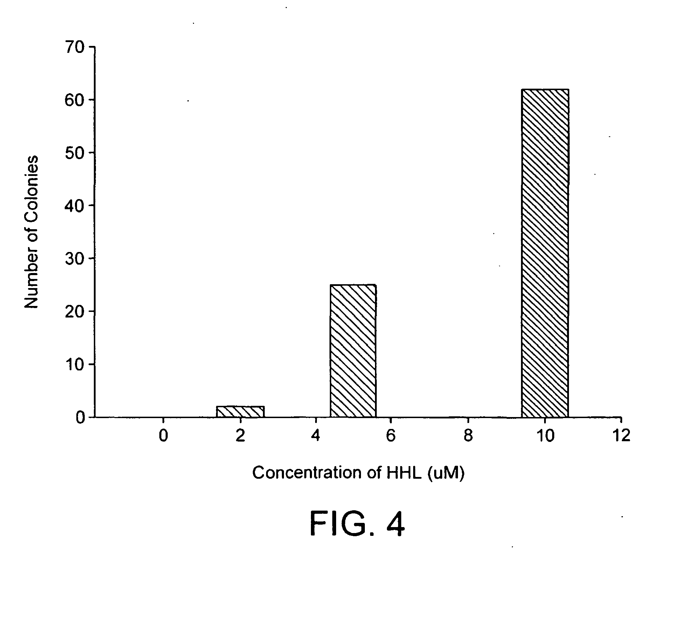

Many motile bacteria employ a system of chemotaxis whereby the cells are able not only to detect the presence of a variety of environmental compounds, but also to monitor concentration gradients of these and to adjust swimming behaviour accordingly. Thus, bacteria will tend to move towards stimuli such as nutrients and away from stimuli that represent unfavourable conditions. It is now known that some bacteria, including *Ps. aeruginosa* exhibit a positive chemotactic response to cell-signalling molecules such as AHLs (see Examples, Fig. 4). This behaviour could be significant in the establishment and development of disease conditions, as the bacteria would tend to move towards each other and towards higher concentrations of signal molecules. This would lead to increases in local cell densities and the earlier establishment of protective biofilm, increases in the local levels of extra-cellular signal molecule concentrations, and a concomitant reduction in the time to switching to a pathogenic phenotype. The antibodies of the present invention and disclosure can therefore be applied to blocking the chemotactic response of bacteria to their own and other species' signal molecules.

Bacterial signalling molecules are being discovered in every organism for which they are searched. It seems to be a ubiquitous system, applicable to every species. The main differences are that all gram negative (gram -ve) bacteria use homoserine lactone-based molecules, and gram positive (gram +ve) bacteria use (modified) small peptides. Previous work in this field has concentrated on mimicking signal molecules with ones that are recognised but that do not function, i.e. no pathogenic switching (Suga and Smith, 2003), or on blocking the various receptor systems. The disadvantages of these methods are principally that resistance can be developed to the mimic or block and the "real" signal molecule is still present and will compete for binding. Furthermore, signal molecule mimics must first enter the bacteria to contact and bind the cell's receptors. In addition, some bacterial signalling molecules e.g. acyl-homoserine lactones are virulence factors in their own right, and can directly cause immuno-suppression of the host (i.e. patient). The present invention provides for methods using antibodies that target the actual signal molecule in the extra-cellular environment rather than the cell itself. This approach has a key and important advantage over all previous efforts in the field in that the bacteria will not recognise that they are being attacked, they will simply detect that that they are alone. There will not be any selective pressure for resistance. The methods of the present invention are further advantageous in that they provide a means to inhibit biofilm formation and as such increase the effectiveness of existing anti-microbial agents such as antibiotics, for which emerging resistance is becoming a serious global concern.

In the present disclosure, the antibody may be a polyclonal antibody. Alternatively, the antibody may be a monoclonal antibody. The antibody may be a single chain antibody (scAb) or an antibody fragment. The antibody fragment may be a single chain antibody (scAb) or a single domain fragment. The antibody may be human antibody or the antibody may be a humanised antibody construct.

In certain disclosed embodiments, the antibodies may be single-chain antibodies (scAbs), such as G3H5, G3B12, G3G2 and/or G3H3 deposited as NCIMB-41167, NCIMB-41168, NCIMB-41169, NCIMB-41170, respectively. The antibody G3B12 is also referred to as Hap 2 and antibody G3G2 is also referred to as Hap 5.

Antibodies according to the present disclosure can, as discussed above, be polyclonal antibodies or monoclonal antibodies. Polyclonal antibodies can be raised by stimulating their production in a suitable animal host (e.g. a mouse, rat, guinea pig, rabbit, sheep, chicken, goat or monkey) when the antigen is injected into the animal. If necessary an adjuvant may be administered together with the antigen. The antibodies can then be purified by virtue of their binding to antigen or as described further below. Monoclonal antibodies can be produced from hybridomas. These can be formed by fusing myeloma cells and B-lymphocyte cells which produce the desired antibody in order to form an immortal cell line. This is the well known Kohler & Milstein technique (Nature 256 52-55 (1975)).

Techniques for producing monoclonal and polyclonal antibodies which bind to a particular protein are now well developed in the art. They are discussed in standard immunology textbooks, for example in Roitt et al, Immunology second edition (1989), Churchill Livingstone, London.

In addition to whole antibodies, the present disclosure includes derivatives thereof which are capable of binding to antigen. Thus the present disclosure includes antibody fragments and synthetic constructs. Examples of antibody fragments and synthetic constructs are given by Dougall et al in Tibtech 12 372-379 (September 1994). Antibody fragments include, for example, Fab, F(ab')₂ and Fv fragments (see Roitt et al [supra]). Fv fragments can be modified to produce a synthetic construct known as a single chain Fv (scFv) molecule. This includes a peptide linker covalently joining V_{H} and V_{L} regions which contribute to the stability of the molecule. The present disclosure therefore also extends to single chain antibodies or scAbs.

Other synthetic constructs include CDR peptides. These are synthetic peptides comprising antigen binding determinants. Peptide mimetics may also be used. These molecules are usually conformationally restricted organic rings which mimic the structure of a CDR loop and which include antigen-interactive side chains. Synthetic constructs also include chimaeric molecules. Thus, for example, humanised (or primatised) antibodies or derivatives thereof are within the scope of the present invention. An example of a humanised antibody is an antibody having human framework regions, but rodent hypervariable regions. Synthetic constructs also include molecules comprising a covalently linked moiety which provides the molecule with some desirable property in addition to antigen binding. For example the moiety may be a label (e.g. a detectable label, such as a fluorescent or radioactive label) or a pharmaceutically active agent.

In order to generate anti-bacterial signal molecule antibodies, it is preferable to conjugate the target molecule, or a suitable derivative, to two different carrier molecules (proteins), though a single conjugated species can be also used. Bacterial signal molecules, in general, are too small to stimulate an immune response *in-vivo,* or to be used directly as a source of antigen for the selection of high affinity antibodies from antibody libraries. Selection of antibodies specific for the cell signalling molecular (hereafter referred to as 'antigen') is carried out in the preferred embodiment using a repertoire (library) of first members of specific binding pairs (sbp), for example a library of antibodies displayed on the surface of filamentous bacteriophage. Any other system that allows for the selection of specific receptors from a library of receptors is also applicable for the methods of the present invention and disclosure. In alternative embodiments signal molecule-specific clones can be selected from a panel of antibody secreting hybridoma cell lines generated from an animal immunised with an antigen conjugate. For the purposes of a general illustration the example of a library of antibody binding sites displayed on phage particles will be used.

A conjugate comprising an antigen coupled to a suitable carrier molecule, which can be a protein, a peptide or any natural or synthetic compound or material (referred to hereafter as 'conjugate-1') is immobilised onto a suitable solid support such as an 'immunotube' or microtitre plate, and the uncoated surface blocked with a nonspecific blocking agent such as dried milk powder. Suitable conjugate molecules can include, but are not limited to proteins such as bovine serum albumin (BSA), Keyhole Limpet Haemocyanin (KLH), Bovine Thyroglobulin (TG), Ovalbumin (Ova), or non-proteins such as biotin. The only restriction on the selection of the conjugate molecule is that it be immobilisable in some way and for immunisation is large enough to elicit an immune response.

A library of first members of specific binding pairs (sbp's) ('the library') is applied to the immobilised conjugate and incubated for sufficient time for sbp members recognising conjugate-1 to bind. Phage not recognising the conjugate are removed by stringent washing. Phage that remain bound are eluted, for example with triethylamine or other suitable reagent, into a buffer solution to restore neutral pH. Recovered phage particles are then infected into a suitable host organism, e.g. E. *coli* bacteria, and cultured to amplify numbers of each selected member and so generate a second 'enriched' library. The process is then repeated using the enriched library to select for phage-antibodies ('phage') recognising the antigen conjugated to a second carrier protein (conjugate-2).

Additional rounds are performed as required, the selection process being altered to favour selection of those sbp members recognising the free form of the antigen. Phage are selected against antigen conjugates as described previously, using initially conjugate-1, and alternating with conjugate-2 (where available) for each subsequent round. Bound phage are eluted by incubating with a solution of free antigen, or antigen conjugated to small soluble selectable moieties, e.g. biotin, for sufficient time for sbp members with higher affinity for the bound form of the antigen to dissociate from the immobilised conjugate. Those phage eluted with free antigen are infected into E. *coli* cells for amplification and re-selection, and those remaining bound to the immobilised antigen discarded. Alternatively, but less preferably, all antibodies binding to conjugate may be eluted e.g. with low pH.

Individual (monoclonal) phage clones from each round of selection are screened for desired binding characteristics. This can be performed by a variety of methods that will be familiar to those with ordinary skill in the art, depending on requirements, including such techniques as SPR (Surface Plasmon Resonance) and ELISA (Enzyme Linked Immuno-Sorbant Assay). Selection criteria will include the ability to bind preferentially to the free soluble form of the antigen in the presence of conjugated derivatives.

In the preferred embodiment of the invention and disclosure, antibodies will be generated from a naïve human antibody phage display library (McCafferty et al., 1990; and as described in WO 92/01047). Thus the antibodies can be used for administering to patients without eliciting an immune response. In other embodiments a library can be constructed from an animal pre-immunised with one or more conjugates of an AHL and a suitable carrier molecule. A further alternative is the generation of hybridoma cell lines from an animal immunised as described above. In the latter two cases it is preferable that steps be taken to reduce the immunogenicity of resulting antibodies, for example by creating host animal-human chimaeric antibodies, or "humanisation" by CDR grafting onto a suitable antibody framework scaffold. Other methods applicable will include the identification of potential T-cell epitopes within the antibody, and the subsequent removal of these e.g. by site-directed mutagenesis (de-immunisation). In a further embodiment the antibody can be engineered to include constant regions from different classes of human immunoglobulin (IgG, IgA, etc.) and produced as a whole antibody molecule in animal cells. In particular these approaches are desirable where the antibodies are to be used therapeutically. The use of secretory IgA isotype antibodies may be preferable where intra-nasal/aerosol application is envisaged for example in the treatment of *Ps. aeruginosa* infections of cystic fibrosis patients.

For the present disclosure, the antibody may be monoclonal or polyclonal. The antibodies may be human or humanised. Antibody fragments or derivatives, such as Fab, F(ab').sup.2 (also written as F(ab')₂), Fv, or scFv, may be used, as may single-chain antibodies (scAb) such as described by Huston et al. (Int. Rev. Immunol. 10: 195-217, 1993), domain antibodies (dAbs), for example a single domain antibody, or antibody-like single domain antigen-binding receptors. In addition to antibodies, antibody fragments and immunoglobulin-like molecules, peptidomimetics or non-peptide mimetics can be designed to mimic the binding activity of antibodies and inhibit or prevent biofilm formation by bacteria.

After the preparation of a suitable antibody, it may be isolated or purified by one of several techniques commonly available (for example, as described in Antibodies: A Laboratory Manual, Harlow and Lane, eds. Cold Spring Harbor Laboratory Press (1988)). Generally suitable techniques include peptide or protein affinity columns, HPLC or RP-HPLC, purification on Protein A or Protein G columns, or combinations of these techniques. Recombinant antibodies can be prepared according to standard methods, and assayed for specificity using procedures generally available, including ELISA, dot-blot assays etc.

The bacteria may be a gram negative bacteria species or a gram positive bacteria species. The bacteria can be selected from the group consisting of *Actinobacillus actinomycetemcomitans, Acinetobacter baumannii, Bordetella pertussis, Brucella* sp., *Campylobacter* sp., *Capnocytophaga* sp., *Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Citrobacter* sp., *Enterobacter* sp., *Escherichia coli, Klebsiella pneumoniae, Proteus* sp., *Salmonella enteriditis, Salmonella typhi, Serratia marcescens, Shigella* sp., *Yersinia enterocolitica, Yersinia pestis, Neisseria gonorrhoeae, Neisseria meningitidis, Moraxella catarrhalis, Veillonella* sp., *Bacteroides fragilis, Bacteroides* sp., *Prevotella* sp., *Fusobacterium* sp., *Spirillum minus, Aeromonas* sp., *Plesiomonas shigelloides, Vibrio cholerae, Vibrio parahaemolyticus, Vibrio vulnificus, Acinetobacter* sp., *Flavobacterium* sp., *Pseudomonas aeruginosa, Burkholderia cepacia, Burkholderia pseudomallei, Xanthomonas maltophilia, Stenotrophomonas maltophila, Staphylococcus aureus, Bacillus spp., Clostridium spp.. and Streptococcus spp.*

The methods and uses of this aspect of the invention may further comprise the administration of an antibiotic. The antibiotic may be a β-lactam antibiotic, such as a penicillin or a penicillin derivative, kanamycin, ampicillin, chloramphenicol, tetracycline, fluoroquinolone, gentamicin, imipenem, and/or carbenicillin, or combinations thereof

According to the disclosure, there is also provided a method for the prevention or inhibition of the formation of a biofilm by a population of bacteria in a subject, the method comprising administration of an antibody as defined above in relation to a use of the first aspect of the invention.

Such methods may further comprise the administration of an antibiotic. The administration of the antibiotic may be at the same time as the administration of the antibody, or it may be prior to, or after said administration of said antibody. The methods are equally applicable to human or veterinary medicine.

Embodiments in accordance with this aspect also extend to the use of an antibody as defined above in relation to the first aspect of the invention in the preparation of a medicament for the prevention or inhibition of the formation of a biofilm by a population of bacteria

According to a second aspect of the invention, there is provided a kit of parts comprising an antibody as defined above in relation to the first aspect of the invention and an antibiotic for separate, subsequent or simultaneous administration for diminishing an established biofilm produced by a population of bacteria. Suitably, such kits will contain instructions for use in the present invention.

According to a third aspect of the invention, there is provided the use of an antibody as defined above in relation to the first aspect of the invention in the preparation of a medicament for diminishing an established biofilm produced by a population of bacteria.

According to a fourth aspect of the invention, there is provided an ex vivo method of diminishing an established biofilm formed by a population of bacteria, said method comprising the administration to the population of bacteria an antibody to the free form of a homoserine lactone signal molecule secreted by bacteria, wherein the antibody is the single chain antibody (scAb) G3B12 deposited as NCIMB - 41168.

According to the disclosure, there is provided as defined herein an antibody to a lactone or lactone-derived signal molecule secreted by bacteria for use in the prevention or inhibition of the formation of a biofilm by a population of bacteria.

Methods and uses in accordance with the present invention may involve the formulation of antibodies described herein, and optionally other pharmaceutically active substances, such as antibiotics, as pharmaceutical compositions. Such compositions may be prepared by any method known in the art of pharmacy, for example by admixing the active ingredient with a carrier(s), diluent (s) or excipient(s) under sterile conditions.

The antibody may be supplied as part of a sterile, pharmaceutical composition which will normally include a pharmaceutically acceptable carrier. This pharmaceutical composition may be in any suitable form, (depending upon the desired method of administering it to a patient).

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions)

Suitable excipients for tablets or hard gelatine capsules include lactose, maize starch or derivatives thereof, stearic acid or salts thereof. Suitable excipients for use with soft gelatine capsules include for example vegetable oils, waxes, fats, semi-solid, or liquid polyols etc. For the preparation of solutions and syrups, excipients which may be used include for example water, polyols and sugars. For the preparation of suspensions oils (e.g. vegetable oils) may be used to provide oil-in-water or water in oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3 (6), page 318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

The antibodies (or equivalent) of the present invention and disclosure could be administered to treat bacterial infection, or used as a preventative measure for those at high risk of infection. In the case where infection already exists, the antibodies may be administered alone or in combination with anti-bacterial antibodies or antibiotics or other anti-microbial treatments. Administration of such antibodies in conjunction with other therapies may allow the use of shorter courses or lower doses of therapeutics, so decreasing the risk of resistance arising and improving patient compliance.

Dosages of the pharmaceutical compositions can vary between wide limits, depending upon the disease or disorder to be treated, the age and condition of the individual to be treated, etc: and a physician will ultimately determine appropriate dosages to be used.

Such compositions may be formulated for human or for veterinary medicine. The present application should be interpreted as applying equally to humans as well as to non-human animals, unless the context clearly implies otherwise. The treatment may be prophylactic or may be in respect of an existing condition. The treatment may also be used to enhance the effectiveness of existing treatments.

The composition may be provided in unit dosage form, and can be provided in a sealed container and may be provided as part of a kit. Such a kit of parts would normally (although not necessarily) include instructions for use. It may include a plurality of said unit dosage forms.

The methods and uses of the invention can be applied to short or long-term, acute or chronic illness/disease caused by bacteria. In a preferred embodiment, the methods and uses of the invention may be directed against an infection caused by the pathogen *Pseudomonas aeruginosa* which is of particular concern with patients suffering from cystic fibrosis. Furthermore, as the methods and uses of the invention are directed particularly at bacterial cell signalling molecules, and not primarily at the bacterial cells themselves, there will be no selective pressure exerted on bacterial populations to develop resistance to the treatments described.

The antibody may be administered to infected patients in order to modulate and reduce bacterial infection by reducing biofilm formation. This can include inhalation of the antibody in an aerosol by cystic fibrosis patients to increase life expectancy or topical application to wounds.

In yet another embodiment conjugates of cell signalling molecules to immunogenic proteins can be administered to individuals or patients in order to stimulate an immune response against the lactone signalling molecule resulting in the generation of neutralising antibodies.

In yet another embodiment alternative methods can be applied to the removal of bacterial cell-cell signalling molecules from the blood of a patient with a view to reducing biofilm formation amongst infecting micro-organisms, thus reducing virulence and causing the bacteria to have increased susceptibility to bactericidal agents and to host defence mechanisms. This can be achieved with other natural receptors (such as antibodies or fragments thereof) or molecules based on natural molecules that bind to said lactone signal molecules. Alternatively non-natural receptors can be applied such as molecularly imprinted polymers (MIPs). This class of receptor have already been shown to be able to bind specifically to small molecular weight bio-molecules such as drugs (Hart et al., 2000) and steroids (Whitcombe et a.,1995; Ramstrom et al., 1996; Rachkov et al., 2000).

In yet another embodiment the receptor may have catalytic or enzymatic activity, and be able to convert the lactone cell signalling molecule into a form that is no longer recognised by the target organism.

According to the disclosure, there is also provided an antibody as defined herein for use in inhibiting or preventing the chemotactic responses of bacteria to cell-signalling molecules. Such uses extend to methods of inhibiting or preventing the chemotactic responses of bacteria to cell-signalling molecules, the method comprising the step of administering a composition comprising an antibody as defined herein to said population of bacteria.

Preferred features for the second and subsequent aspects of the invention are as for the first aspect *mutatis mutandis.*

Other objects, features and advantages of the present invention, including but not limited to related applications in animal hosts, will be apparent to those skilled in the art after review of the specification and claims of the invention.

It will be apparent to those of ordinary skill in the art that the compositions and methods disclosed herein may have application across a wide range of organisms in inhibiting biofilm formation, and modulating or treating conditions resulting from infection. The compositions and methods of the present invention are described with reference to *Pseudomonas aeruginosa,* but it is within the competence of one of ordinary skill in the art to apply the objects herein to other species.

The invention will now be further described by reference to the non-limiting example and figures detailed below.

### Description of Figures

Figure 1 shows the effect on the development of biofilm growth of *Ps. aeruginosa* PA14 of different concentrations of the anti-AHL single-chain antibody fragment (scAb) Hap-2 (•) compared to controls in which there were either no antibody (□) or no bacterial cells (O) present.
Figure 2 shows effects of Hap-2 scAb (•), tetracycline (▼), Hap-2 + tetracycline (O) on biofilm formation at 6 hours compared to controls with bacteria only (■) and PBS alone (□).
Figure 3 shows a time course for the inhibition of biofilm by Hap-2 (□) compared to no antibody (□), antibody alone (□) and no-bacteria (□) controls.
Figure 4 shows the movement of motile *Ps. aeruginosa* towards different concentrations of the quorum-sensing signal molecule hexanoyl homoserine lactone (HHL).

### Examule 1: Generation of anti-AHL antibodies.

A naïve human antibody phage display library was screened against conjugates of the acyl-homoserine lactone dDHL (dodecanoyl homoserine lactone). Briefly, a derivative of dDHL including a carboxyl group at the end of the acyl chain was conjugated to the carrier proteins Bovine Serum Albumin (BSA) and Bovine Thyroglobulin (TG) using well known chemistry. The antibody library was screened (panned) against each conjugate alternately for three rounds, with those phage binding to conjugate being isolated, amplified, and used for the subsequent round. After the first round, all binding phage were recovered and amplified. During the second and third rounds, bound phage were eluted from the immobilised conjugate by incubation with a solution of free soluble native dDHL. Monoclonal phage antibodies from round three were screened initially for binding to both AHL conjugates, and to carrier protein alone. Those clones binding only to the conjugated antigen were further screened for the ability to bind to free dDHL by competitive binding ELISA. A clone designated Hap 2 was isolated that could be inhibited in binding to dDHL-conjugate in the presence of free dDHL or free BHL (N-butyl-homoserine lactone).

### Determining biofilm production

The formation of biofilms by *Pseudomonas aeruginosa* was measured by assessing the ability of growing cells to adhere to the surface of polypropylene 96-well microtitre plates according the methods described by Conway et al., 2002. Ps *aeruginosa* strain PA14 was inoculated into 5 ml LB broth and incubated overnight at 37°C. The following day, the bacteria were inoculated to 1% into a 96-well tissue culture plate containing 100 µl/well LB broth, and incubated overnight in a humidified environment at 37°C. The use of a minimal medium (LB) prevents the formation of biofilm.

The plate was centrifuged at 2,500 rpm for 10 min, and the supernatant aspirated off taking care not to disturb the pelleted cells. Cells were then resuspended in-situ with 100 µl/well brain/heart infusion broth. The cultures were incubated at 37°C for 2 h. The medium was removed and the wells washed three times with 200 µl/well dH₂O to remove any remaining planktonic cells, care being taken to avoid disturbing any biofilm that had developed. Attached cells (biofilm) were stained by addition of 125 µl/we// 1% solution of crystal violet followed by incubation at room temperature for 15 min. Excess stain was then removed by washing the plate thoroughly with dH₂O. The crystal violet stain, which is a measure of the extent of the biofilm produced, was recovered with 200 µl /well 95% ethanol. The absorbance of 125 µl ethanol/crystal violet was measured at 590 nm.

### Inhibition of biofilms

Biofilm assays were carried out as described above. When pelleted bacteria were resuspended in brain/heart infusion broth, a further addition was made of 100 µl/well Hap 2 single-chain antibody fragment (scAb) in PBS or PBS alone.

### i) Titration of biofilm inhibition.

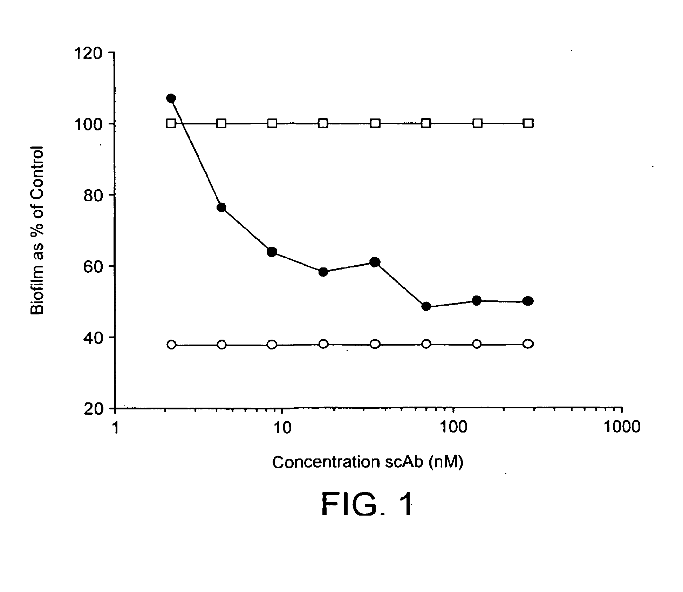

A dilution series of Hap-2 anti-AHL scAb was set up in duplicate wells to determine the effect of antibody concentration on the establishment of biofilm by *Ps. aeruginosa* PA14. Incubation was continued as described above for 2 h, and the effect of Hap 2 addition on biofilm formation determined by the amount of crystal violet stain fixed at various scAb concentrations. Control experiments were conducted in which either scAb was replaced by PBS, or bacteria were also omitted to assess how much stain was passively adsorbed onto the plates. The results clearly demonstrate that the addition of Hap-2 scAb causes a concentration dependant inhibition of biofilms (Figure 1). A concentration of 70 nM scAb is sufficient to reduce biofilm by approximately 80%.

### ii) The effect of antibody and tetracycline.

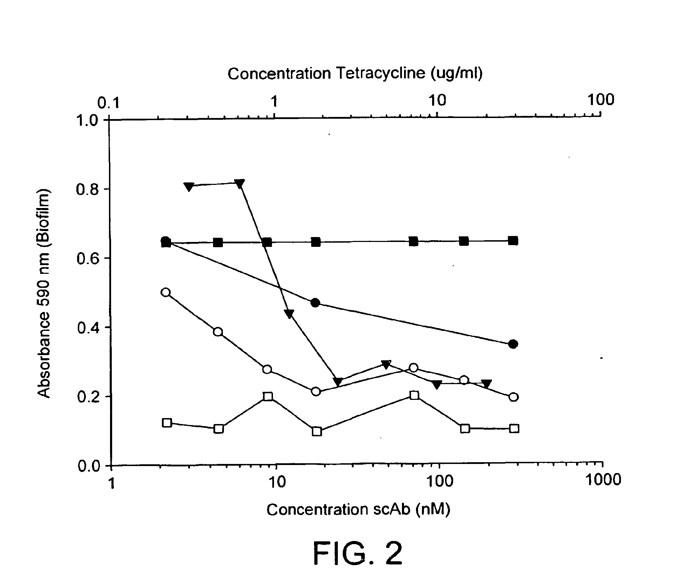

The antibiotic tetracycline is known to inhibit biofilm formation amongst *Pseudomons* sp. The biofilm assay was carried out essentially as described above, however incubation time for cultures in the presence of biofilm inhibitors was extended to 6 hours. In addition to a dilution series of scAb, duplicate wells were also treated with a dilution series of tetracycline. A further set of wells included both scAb and tetracycline. The results indicate that while both scAb and tetracycline are effective at reducing biofilm formation over an extended period, combining the two provides an additive effect at low concentrations (Figure 2).

### iii) Biofilm inhibition time-course.

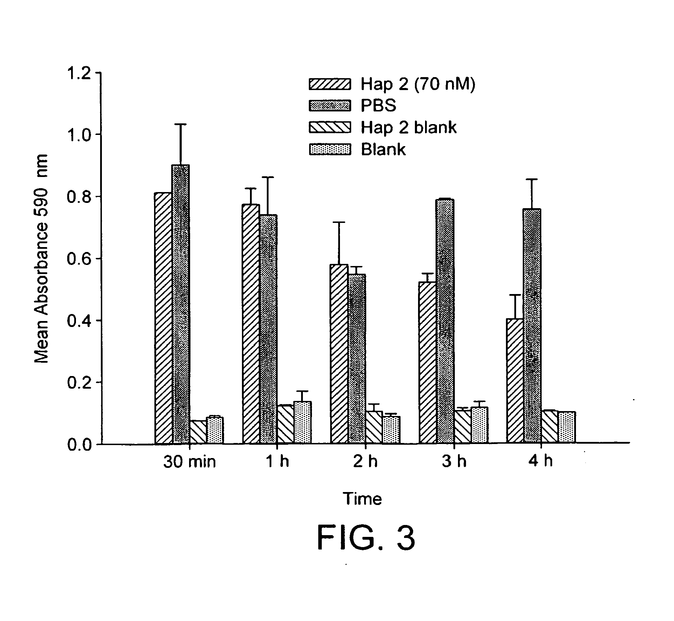

The effect of scAb addition to biofilm formation over time was assessed by performing the assay over a 4 hour period and taking sample readings at several time points. The anti-AHL scAb Hap-2 at 70 nM concentration was compared with cultures in which scAb was not present, and the effects scAb and PBS alone on crystal violet adsorption included as controls (Figure 3). The results suggest that the bacterial cells adhere very quickly to surfaces when grown in rich media, and that Hap-2 progressively diminishes the rapidly established biofilm with time.

### Example 2: Establishing a link between Quorum sensing and chemotaxis.

An assay was carried out to determine whether or not *Ps. aeruginosa* is able not only to respond to the presence of AHL cell-signalling molecules by altering it's phenotype, but is also able to actively seek out fellow bacteria by directional detection of AHL molecules and movement towards the source.

Four wells were cut into LB agar plates near the edge of the plates, and 90° from each other radially using a core-borer. One hundred microlitres of HHL (hexanoyl homoserine lactone) at various concentrations was applied to each of three of the wells. PBS was added to the fourth as a control. Twenty microlitres of a 1% inoculum of an overnight culture of *Ps. aeruginosa* PA14 was spotted into the centre of the plates, equi-distant from each of the wells, and the plates incubated overnight at 37°C.

In addition to the central spot where the bacteria were applied to the plate, a number of colonies were observed at various distances from the centre. These developed from motile cells that had migrated across the agar surface before settling and growing in a sessile manner. The plates were marked into four equal quadrants, such that each well was equi-distant from each adjacent quadrant. The numbers of colonies lying in each quadrant was counted. The results show that *Ps. aeruginosa* does exhibit a chemotactic response to the presence of HHL, the number of cells swimming towards the HHL being proportional to the concentration applied to the well, and therefore to the strength of the concentration gradient (Figure 4).

### References

6,309,651
WO 01/26650
WO 01/74801
WO 92/01047
GB2003/003529
Shih and Huang, 2002 J. Antimicrobial Chemotherapy 49: 309-314
Wozniak et al., 2003 Proc. Natl. Acad. Sci. USA 100 (13): 7907-7912
Harrisson-Baestra et. al., 2003 Dermatol. Surg. 29 (6): 631-635
Williams et al., 1996 Microbiol-UK 142: 881-888
Stintzi et al., 1998 FEMS Microbiol Lett. 166 (2): 341-345
Glessner et al., 1999 J. Bacteriol. 181 (5): 1623-1629
Brint and, Ohman 1995 J. Bacteriol. 177 (24): 7155-7163
Reimmann et al., 1997 Mol. Microbiol. 24 (2): 309-319
Winzer et al., 2000 J. Bacteriol. 182 (22): 6401-6411
Gambello and Iglewski 1991 J. Bacteriol. 173 (9): 3000-3009
Latifi et al., 1995 Mol. Microbiol 17 (2): 333-343
Passador et al., 1993 Science 260: 1127-1130
Pearson et al., 1994 Proc. Natl. Acad. Sci. USA. 91 (1): 197-201
Winson et al., 1995 Proc. Natl. Acad. Sci. USA. 92 (20): 9427-9431
Pesci et al., 1997 J. Bacteriol. 179 (10): 3127-3132
Toder et al., 1991 Mol. Microbiol. 5 (8): 2003-2010
Gambello et al., 1993 Infect. Immun. 61 (4): 1180-1184
Ochsner et al., 1994 J. Bacteriol. 176, 2044-2054
Pearson et al., 1995 Proc. Natl. Acad. Sci. USA 92 (5) 1490-1494
Latifi et al., 1996 Mol. Microbiol 21 (6): 1137-1146
Winzer et al., 2000 J. Bacteriol. 182 (22): 6401-6411
Pesci et al, 1999 Proc. Natl. Acad. Sci. USA 96: 11229-11234
Burns et al., 2001 J. Infect. Dis. 183: 444-452
Singh et al., 2000 Nature 407: 762-764
Wu et al., 2000 Microbiology 146: 2481-2493
Wu et al., 2004 Microbes and Infection 6: 34-37
Manefield et al., 1999 Microbiol. UK 145: 283-291
Tepletski et al., 2000 Mol. Plant Microbe. Interact., 13: 637-648
Suga and Smith, 2003 Current Opinion in Chemical Biology 7: 586-591
Kohler and Milstein, 1975 Nature 256: 495-497
Roitt et al., Immunology second edition (1989), Churchill Livingstone, London.
Dougall et al., 1994 TibTech 12: 372-379
McCafferty et al., 1990 Nature 348: 552-554
Huston et al., 1993 Int. Rev. Immunol., 10: 195-217
Antibodies: A Laboratory Manual, Harlow and Lane, eds. Cold Spring Harbor Laboratory Press (1988)
Hart et al., 2000 J. Am. Chem. Soc., 122, 460-465
Whitcombe et al., 1995 J. Am. Chem. Soc., 117, 7105-7111
Ramstrom et al., 1996 Chem. & Biol., 3, 471-477
Rachkov et al., 2000 Anal. Chim. Acta. 405, 23-29
Conway et al., 2002 J. Bacteriol. 184 (20): 5678-5685

## Claims

1. An antibody to the free form of a homoserine lactone signal molecule secreted by bacteria for use in diminishing an established biofilm produced by a population of bacteria, wherein the antibody is the single chain antibody (scAb) G3B12 deposited as NCIMB-41168.

2. An antibody for use as claimed in claim 1, in which the bacteria is selected from the group consisting of *Actinobacillus actinomycetemcomitans, Acinetobacter baumannii, Bordetella pertussis, Brucella* sp., *Campylobacter* sp., *Capnocytophaga* sp., *Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Citrobacter* sp., *Enterobacter* sp., *Escherichia coli, Klebsiella pneumoniae, Proteus* sp., *Salmonella enteriditis, Salmonella typhi, Serratia marcescens, Shigella* sp., *Yersinia enterocolitica, Yersinia pestis, Neisseria gonorrhoeae, Neisseria meningitidis, Moraxella catarrhalis, Veillonella* sp., *Bacteroides fragilis, Bacteroides* sp., *Prevotella* sp., *Fusobacterium* sp., *Spirillum minus, Aeromonas* sp., *Plesiomonas shigelloides, Vibrio cholerae, Vibrio parahaemolyticus, Vibrio vulnificus, Acinetobacter* sp., *Flavobacterium* sp., *Pseudomonas aeruginosa, Burkholderia cepacia, Burkholderia pseudomallei, Xanthomonas maltophilia, Stenotrophomonas maltophila, Staphylococcus aureus, Bacillus spp., Clostridium spp.. and Streptococcus spp.*

3. A kit of parts comprising an antibody as defined in claim 1 and an antibiotic for separate, subsequent or simultaneous administration for diminishing an established biofilm produced by a population of bacteria.

4. The use of an antibody as defined in claim 1 in the preparation of a medicament for diminishing an established biofilm produced by a population of bacteria.

5. An *ex vivo* method of diminishing an established biofilm formed by a population of bacteria, said method comprising the administration to the population of bacteria an antibody to the free form of a homoserine lactone signal molecule secreted by bacteria, wherein the antibody is the single chain antibody (scAb) G3B12 deposited as NCIMB-41168.

## Patentansprüche

1. Antikörper gegen die freie Form eines Homoserinlacton-Signalmoleküls, das durch Bakterien sekretiert wird, zur Verwendung bei der Verminderung eines entwickelten Biofilms, der durch eine Population von Bakterien produziert wird, wobei der Antikörper der Einzelkettenantikörper (scAb) G3B12 ist, der als NCIMB-41168 hinterlegt ist.

2. Antikörper zur Verwendung, wie in Anspruch 1 beansprucht, wobei die Bakterien ausgewählt sind aus der Gruppe, bestehend aus *Actinobacillus actinomycetemcomitans, Acinobacter baumannii, Bordetella pertussis, Brucella* sp., *Campylobacter* sp., *Capnocytophaga* sp., *Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Citrobacter* sp., *Enterobacter* sp., *Escherichia coli, Klebsiella pneumoniae, Proteus* sp., *Salmonella enteriditis, Salmonella typhi, Serratia marcescens, Shigella* sp., *Yersinia enterocolitica, Yersinia pestis, Neisseria gonorrhoeae, Neisseria meningitidis, Moraxella catarrhalis, Veillonella* sp., *Bacteroides fragilis, Bacteroides* sp., *Prevotella* sp., *Fusobacterium* sp., *Spirillum minus, Aeromonas* sp., *Plesiomonas shigelloides, Vibrio cholerae, Vibrio parahaemolyticus, Vibrio vulnificus, Acinetobacter* sp., *Flavobacterium* sp., *Pseudomonas aeruginosa, Burkholderia cepacia, Burkholderia pseudomallei, Xanthomonas maltophilia, Stenotrophomonas maltophila, Staphylococcus aureus, Bacillus spp., Clostridium spp.* und *Streptococcus spp.*

3. Kit von Komponenten, umfassend einen Antikörper, wie in Anspruch 1 definiert, und ein Antibiotikum zur getrennten, nachfolgenden oder gleichzeitigen Verabreichung zur Verringerung eines entwickelten Biofilms, der durch eine Bakterienpopulation produziert wurde.

4. Verwendung eines Antikörpers, wie in Anspruch 1 definiert, bei der Herstellung eines Medikaments zur Verringerung eines entwickelten Biofilms, der durch eine Bakterienpopulation produziert wurde.

5. Ex-vivo-Verfahren zur Verringerung eines entwickelten Biofilms, der durch eine Bakterienpopulation gebildet wurde, wobei das Verfahren die Verabreichung eines Antikörpers gegen die freie Form eines Homoserinlacton-Signalmoleküls, das durch Bakterien sekretiert wird, an die Bakterienpopulation umfasst, wobei der Antikörper der Einzelkettenantikörper (scAb) G3B12 ist, der als NCIMB-41168 hinterlegt ist.

## Revendications

1. Anticorps vis-à-vis de la forme libre d'une molécule signal d'homosérine lactone sécrétée par une bactérie, pour une utilisation dans la réduction d'un biofilm adhérent produit par une population de bactéries, l'anticorps étant l'anticorps à chaîne simple (scAb) G3B12 déposé sous la référence NCIMB-41168.

2. Anticorps pour une utilisation telle que revendiquée dans la revendication 1, la bactérie étant sélectionnée dans le groupe consistant en *Actinobacillus actinomycetemcomitans, Acinetobacter baumannii, Bordetella pertussis, Brucella* sp., *Campylobacter* sp., *Capnocytophaga* sp., *Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Citrobacter* sp., *Enterobacter* sp., *Escherichia coli, Klebsiella pneumoniae, Proteus* sp., *Salmonella enteriditis, Salmonella typhi, Serratia marcescens, Shigella* sp., *Yersinia enterocolitica, Yersinia pestis, Neisseria gonorrhoeae, Neisseria meningitidis, Moraxella catarrhalis, Veillonella* sp., *Bacteroides fragilis, Bacteroides* sp., *Prevotella* sp., *Fusobacterium* sp., *Spirillum minus, Aeromonas* sp., *Plesiomonas shigelloides, Vibrio cholerae, Vibrio parahaemolyticus, Vibrio vulnificus, Acinetobacter* sp., *Flavobacterium* sp., *Pseudomonas aeruginosa, Burkholderia cepacia, Burkholderia pseudomallei, Xanthomonas maltophilia, Stenotrophomonas maltophila, Staphylococcus aureus, Bacillus* spp., *Clostridium* spp. *et Streptococcus* spp.

3. Kit composé de plusieurs parties, comprenant un anticorps tel que défini dans la revendication 1 et un antibiotique pour une administration séparée, consécutive ou simultanée pour réduire un biofilm adhérent produit par une population de bactéries.

4. Utilisation d'un anticorps telle que définie dans la revendication 1, dans la préparation d'un médicament destiné à réduire un biofilm adhérent produit par une population de bactéries.

5. Procédé ex *vivo* de réduction d'un biofilm adhérent formé par une population de bactéries, ledit procédé comprenant l'administration à la population de bactéries d'un anticorps vis-à-vis de la forme libre d'une molécule signal d'homosérine lactone sécrétée par une bactérie, l'anticorps étant l'anticorps à chaîne simple (scAb) G3B12 déposé sous la référence NCIMB-41168.
